# EUROPEAN PATENT APPLICATION

(11) **EP 0 728 720 A1**
(43) Date of publication of application: **28.08.1996**
(21) Application number: 95900294.0
(22) Date of filing: 11.11.1994
(51) Int. Cl.: C07C 19/08, C07C 17/23, C07C 19/10, C07C 17/20, C07C 19/01, C07C 17/278, B01J 23/40

(54) **PROCESS FOR PRODUCING 1,1,1,3,3-PENTAFLUOROPROPANE**

(30) Priority: 12.11.1993 JP 283099/93
(71) Applicant: DAIKIN INDUSTRIES, LIMITED, Osaka-shi Osaka 530 (JP)
(72) Inventor: AOYAMA, Hirokazu, Yodogawa Works of Daikin Ind., Osaka 566 (JP); YAMAMOTO, Akinori, Yodogawa Works of Daikin Ind., Osaka 566 (JP)
(74) Representative: Hansen, Bernd, Dr. Dipl.-Chem.
(86) International application number: JP9401915
(87) International publication number: WO9513256

(57) **Abstract**

1,1,1,3,3-pentafluoropropane is prepared at a high yield and a high selectivity by reducing 3-chloro-1,1,1,3,3-pentafluoropropane with hydrogen in a gas phase in the presence of a noble metal catalyst.

## Description

### FIELD OF THE INVENTION

The present invention relates to a process for preparing 1,1,1,3,3-pentafluoropropane which is a useful compound as a substitute for CFC and HCFC which are used as coolants, blowing agents, cleaners, and so on.

### PRIOR ART

US Patent No. 2,942,036 discloses a process for preparing 1,1,1,3,3-pentafluoropropane by reducing 2,2,3-trichloro-1,1,1,3,3-pentafluoropropane in the presence of a palladium catalyst supported on activated carbon. However, this process is not suitable for the industrial production, since a yield of intended 1,1,1,3,3-pentafluoropropane is as low as about 60 % at best, while a large amount of 1,1,1,3,3-pentafluoropropene is by-produced.

### SUMMARY OF THE INVENTION

An object of the present invention is to provide a process for preparing 1,1,1,3,3-pentafluoropropane in a high yield by reducing 3-chloro-1,1,1,3,3-pentafluoropropane.

According to the present invention, there is provided a process for preparing 1,1,1,3,3-pentafluoropropane comprising reducing 3-chloro-1,1,1,3,3-pentafluoropropane with hydrogen in a gas phase in the presence of a noble metal catalyst.

According to this process, desired 1,1,1,3,3-pentafluoropropane is obtained in a high yield and a high selectivity.

### DETAILED DESCRIPTION OF THE INVENTION

Examples of the noble metal catalyst to be used in the process of the present invention are palladium, platinum, rhodium, ruthenium, and so on. Among them, palladium, platinum and rhodium are preferred, and palladium is particularly preferred.

The noble metal catalyst may be supported on various carriers. As the carrier, any conventional carrier such as activated carbon, alumina, silica gel, titanium oxide, and zirconia may be used. Among them, activated carbon is preferred.

A particle size of the carrier has little influence on the reaction. Preferably, it is from 0.1 to 100 mm.

A supported amount of the catalyst is selected from the wide range between 0.05 wt. % and 10 wt. %. An amount of 0.5 to 5 wt. % is preferred because of the easy availability and cost.

A reaction temperature is usually from 100 to 350°C, preferably from 150 to 300°C. When the reaction temperature is lower than 100°C, a conversion may be decreased significantly, while when the reaction temperature is higher than 350°C, by-products may be formed, or an apparatus used in the reaction tends to be damaged.

In the process of the present invention, a ratio of 3-chloro-1,1,1,3,3-pentafluoropropane to hydrogen is such that at least one equivalent of hydrogen is used based on 3-chloro-1,1,1,3,3-pentafluoropropane, and may be changed in a wide range. Usually, hydrogen is used in an amount of 1 to 10 times, preferably 2 to 5 times the stoichiometric amount in relation to 3-chloro-1,1,1,3,3-pentafluoropropane.

Hydrogen may be used in an amount much larger than the stoichiometric amount, for example, 10 moles or more. Excessive hydrogen can be recovered and recycled.

In the process of the present invention, the gas phase reaction may be performed by any one of conventional gas phase reaction systems, such as a fixed bed gas phase reaction, a fluidized bed gas phase reaction, and so on.

Reaction pressure is not limited specifically, and the reaction can be carried out under elevated pressure, reduced pressure or atmospheric pressure. Preferably, the reaction is carried out under the elevated pressure or the atmospheric pressured, since the reaction apparatus becomes complicated under the reduced pressure.

A contact time is usually from 0.1 to 300 seconds, preferably from 1 to 30 seconds.

For example, 3-chloro-1,1,1,3,3-pentafluoropropane as the raw material may be prepared economically in an industrial scale by fluorinating 1,1,1,3,3,3-hexachloropropane with hydrogen fluoride in the presence of a catalyst (see JP-A-5-201892). 1,1,1,3,3,3-hexachloropropane may be obtained by adding carbon tetrachloride to vinylidene chloride (see the above JP publication).

The present invention will be illustrated by the following Examples.

### Example 1

In a stainless steel (SUS 316) reactor having an inner diameter of 7 mm and a length of 150 mm, a palladium catalyst supported on activated carbon in a concentration of 3 wt. % (2.3 cc) was filled, and heated to 200°C with an electric furnace while flowing nitrogen gas therethrough.

After reaching the specific temperature, 3-chloro-1,1,1,3,3-pentafluoropropane and hydrogen were introduced in the reactor at flow rates of 4 cc/min. and 8 cc/min., respectively. The reaction temperature was maintained at 200°C. A generated gas was washed with water and then analyzed by gas chromatography. The results are shown in the following Table.

### Example 2

In the same reactor as used in Example 1, a palladium catalyst supported on activated carbon in a concentration of 3 wt. % (2.3 cc) was filled, and heated to 200°C with an electric furnace while flowing nitrogen gas therethrough.

After reaching the specific temperature, 3-chloro-1,1,1,3,3-pentafluoropropane and hydrogen were introduced in the reactor at flow rates of 3 cc/min. and 9 cc/min., respectively. The reaction temperature was maintained at 200°C. A generated gas was washed with water and then analyzed by gas chromatography. The results are shown in the following Table.

### Example 3

In the same reactor as used in Example 1, a palladium catalyst supported on activated carbon in a concentration of 3 wt. % (2.3 cc) was filled, and heated to 250°C with an electric furnace while flowing nitrogen gas therethrough.

After reaching the specific temperature, 3-chloro-1,1,1,3,3-pentafluoropropane and hydrogen were introduced in the reactor at flow rates of 4 cc/min. and 8 cc/min., respectively. The reaction temperature was maintained at 250°C. A generated gas was washed with water and then analyzed by gas chromatography. The results are shown in the following Table.

### Example 4

In the same reactor as used in Example 1, a palladium catalyst supported on activated carbon in a concentration of 3 wt. % (2.3 cc) was filled, and heated to 150°C with an electric furnace while flowing nitrogen gas therethrough.

After reaching the specific temperature, 3-chloro-1,1,1,3,3-pentafluoropropane and hydrogen were introduced in the reactor at flow rates of 3 cc/min. and 9 cc/min., respectively. The reaction temperature was maintained at 150°C. A generated gas was washed with water and then analyzed by gas chromatography. The results are shown in the following Table.

**Table**

| Example No. | Conversion (%) | Selectivity (%) |
|---|---|---|
| 1 | 95 | 98 |
| 2 | 100 | 97 |
| 3 | 99 | 95 |
| 4 | 92 | 90 |

## Claims

1. A process for preparing 1,1,1,3,3-pentafluoropropane comprising reducing 3-chloro-1,1,1,3,3-pentafluoropropane with hydrogen in a gas phase in the presence of a noble metal catalyst.

2. The process according to claim 1 wherein hydrogen is used in an amount of 1 to 10 equivalents to 3-chloro-1,1,1,3,3-pentafluoropropane.

3. The process according to claim 1, wherein said noble metal catalyst comprises at least one metal selected from the group consisting of palladium, platinum and rhodium.

4. The process according to claim 1, wherein said noble metal catalyst is supported on at least one carrier selected from the group consisting of activated carbon, alumina, silica gel, titanium oxide and zirconia.

5. The process according to claim 4, wherein an amount of said noble metal carried on said carrier is 0.05 to 10 wt. %.

6. The process according to claim 1, wherein the reduction with hydrogen is carried out in a temperature range between 100°C and 350°C.

7. The process according to claim 7, wherein 3-chloro-1,1,1,3,3-pentafluoropropane is obtained by adding carbon tetrachloride to vinylidene chloride to obtain 1,1,1,3,3,3-hexachloropropane, and then fluorinating it with hydrogen fluoride.
